# DEMANDE DE BREVET EUROPEEN

(11) **EP 0 749 732 A1**
(43) Date de publication de la demande: **27.12.1996**
(21) Numéro de dépôt: 96401314.8
(22) Date de dépôt: 18.06.1996
(51) Int. Cl.: A61F 2/16

(54) **Dispositif d'implant intraoculaire pour la correction de l'anisotropie oculaire**

(30) Priorité: 22.06.1995 FR 9507477
(71) Demandeur: W.K. ET ASSOCIES, 75012 Paris (FR)
(72) Inventeur: Weiser, Marc, 75006 Paris (FR)
(74) Mandataire: Dronne, Guy

(57) **Abrégé**

Dispositif d'implant intraoculaire pour la correction de I'anisotropie oculaire et notamment de l'astigmatisme comprenant une partie optique (20, 22) ayant une forme sensiblement circulaire et étant apte à corriger l'anisotropie selon une direction diamétrale et une partie haptique (10, 12). Ladite partie optique et ladite partie haptique sont constituées de deux pièces différentes. Ladite partie haptique comprend une première extrémité apte à coopérer avec la paroi interne de l'oeil, lorsque le dispositif d'implant est disposé dans l'oeil, pour maintenir en position ladite partie optique et une deuxième extrémité (12) apte à coopérer avec la périphérie de ladite partie optique (22) pour définir des moyens de guidage en rotation de ladite partie optique autour de son axe optique, des moyens de solidarisation en translation de ladite partie optique par rapport à ladite partie haptique et des moyens de freinage pour n'autoriser la rotation de ladite partie optique que sous l'effet d'un couple appliqué à ladite partie optique.

## Description

La présente invention a pour objet un dispositif d'implant intraoculaire pour la correction de l'anisotropie oculaire.

Dans l'ensemble du présent texte, par anisotropie oculaire, il faut entendre tous les types d'altération du fonctionnement de l'oeil qui entraîne le fait que, par rapport à l'axe optique de l'oeil, l'image perçue par celui-ci ne présente pas une symétrie de révolution autour de cet axe. En d'autres termes, par ce mot, il faut entendre que selon les différentes directions diamétrales de l'oeil, par rapport à son axe optique, l'image d'un objet reçue par la rétine n'a pas la même dimension, ou bien n'a pas la même focalisation, ce qui entraîne des différences de netteté. Ce type d'altération de la vision se présente en particulier sous la forme de l'astigmatisme qui correspond à une altération des rayons de courbure de la cornée. Comme cela est bien connu, cet astigmatisme peut être "naturel", c'est-à-dire pré-opératoire ou au contraire post-opératoire, c'est-à-dire qu'il résulte de l'effet d'une incision pratiquée sur l'oeil.

On comprend que, pour corriger cette altération de la vision, il est nécessaire de disposer d'un système optique correcteur qui présentera lui-même une anisotropie de correction optique selon les directions radiales par rapport à l'axe optique de ce système correcteur. Des implants intraoculaires, dont les faces sont taillées de manière à introduire cette correction anisotropique de la vue sont en soit bien connus. Dans ce cas, les deux faces de la lentille de l'implant intraoculaire sont des combinaisons de surface sphérique, cylindrique, torique on encore conique.

On comprend que pour la mise en place d'un tel système optique correcteur, il est déterminant que le positionnement angulaire de ce système optique soit parfaitement déterminé par rapport à l'oeil de telle manière que la correction optique compense effectivement le défaut de vision. Ce résultat est obtenu facilement dans le cas où la correction est apportée par l'intermédiaire de paires de lunettes dont les verres ont une position angulaire bien sûr parfaitement déterminée par rapport à l'oeil à corriger.

En revanche, dans le cas d'implants intraoculaires, la situation se présente différemment. On sait en effet qu'un implant intraoculaire est constitué par une partie optique constituée essentiellement par une lentille correctrice et par une partie haptique dont la périphérie coopère avec la paroi interne de l'oeil, par exemple celle du sac capsulaire pour maintenir en position la partie optique de telle façon que l'axe optique de celle-ci coïncide sensiblement avec l'axe optique de l'oeil implanté. Il s'avère que lors de la mise en place de l'implant intraoculaire dans l'oeil, il est délicat de lui donner une orientation angulaire très précise, notamment du fait de l'élasticité nécessaire de la partie haptique qui peut induire un certain couple de rotation de la partie optique après sa mise en place, ce qui a pour effet de modifier la position angulaire. Une autre difficulté consiste dans le fait qu'après la mise en place de l'implant dans l'oeil, la périphérie de la partie haptique peut glisser par rapport à la paroi interne de l'oeil, par exemple, sous l'effet d'un choc reçu par celui-ci. En effet, il s'avère que même dans la chambre antérieure de l'oeil, ou le plus souvent dans le sac capsulaire, l'effet de symphyse capsulaire ou de synéchie qui permet la solidarisation de l'extrémité de la partie haptique sur la paroi de l'oeil ne se développe qu'après un certain temps à partir de la date de l'implantation, ce temps pouvant être de l'ordre de 2 à 3 mois. On comprend également qu'après que ces phénomènes se soient développés, il n'est plus possible de procéder à une nouvelle intervention, pour remettre en place angulairement l'implant intraoculaire. Il s'ensuit que les risques de mauvais positionnement angulaire de l'implant sont très élevés.

Un objet de la présente invention est de fournir un implant intraoculaire permettant la correction de l'anisotropie oculaire qui permette d'obtenir effectivement un positionnement angulaire de l'implant parfaitement déterminé.

Pour atteindre ce but, le dispositif d'implant intraoculaire pour la correction de l'anisotropie oculaire et notamment de l'astigmatisme qui comprend une partie optique ayant une forme sensiblement circulaire et étant apte à corriger l'anisotropie selon une direction diamétrale, se caractérise en ce que ladite partie optique et ladite partie haptique sont constituées de deux pièces différentes, en ce que ladite partie haptique comprend une première extrémité apte à coopérer avec la paroi interne de l'oeil, lorsque le dispositif d'implant est disposé dans l'oeil, pour maintenir en position ladite partie optique et une deuxième extrémité apte à coopérer avec la périphérie de ladite partie optique pour définir des moyens de guidage en rotation de ladite partie optique autour de son axe optique, des moyens de solidarisation en translation de ladite partie optique par rapport à ladite partie haptique selon la direction de l'axe optique, et des moyens de freinage pour n'autoriser la rotation de ladite partie optique que sous l'effet d'un couple appliqué à ladite partie optique ayant une intensité supérieure à une valeur prédéterminée.

On comprend que grâce à l'architecture de cet implant, il est possible, lorsque la partie haptique est devenue solidaire de la paroi interne de l'oeil et notamment du sac capsulaire, ce qui a pour effet d'immobiliser en rotation la partie optique, de procéder à une intervention chirurgicale légère pour provoquer la rotation de la partie optique par rapport à la partie haptique de façon contrôlée pour amener la partie optique dans la position angulaire requise. Grâce aux moyens de freinage, on comprend que cette position angulaire sera maintenue sans aucun risque de modification.

Selon un premier mode de mise en oeuvre de l'invention, la partie optique est constituée par une unique lentille montée rotative dans la partie optique, cette lentille unique présentant une direction diamétrale de correction de l'anisotropie.

Selon un mode perfectionné de réalisation, la partie optique de l'implant est constituée par deux lentilles séparées, dont les axes optiques sont confondus et qui peuvent tourner séparément. Après l'immobilisation de la partie optique, il est possible de procéder chirurgicalement à la modification de la position angulaire des différentes lentilles, afin non seulement d'adapter la direction diamétrale de correction de l'anisotropie oculaire mais également de modifier la puissance de la correction en adaptant la position angulaire relative des deux lentilles.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées sur lesquelles :
- les figures 1a et 1b montrent respectivement en vue de face et de côté un mode de réalisation de la partie haptique dans le cas où l'implant ne comporte qu'une seule lentille ;
- les figures 2a et 2b montrent respectivement en vue de face et en coupe diamétrale une lentille constituant la partie optique de l'implant;
- les figures 3a et 3b montrent respectivement en vue de face et en vue de côté l'implant intraoculaire complet selon un premier mode de réalisation d'un implant comportant une seule lentille ;
- les figures 4a et 4b montrent un deuxième mode de réalisation de l'implant respectivement en vue de face et en coupe diamétrale dans le cas où l'implant comporte deux lentilles séparées ;
- la figure 5 montre en coupe diamétrale une première variante du deuxième mode de réalisation de l'implant comportant deux lentilles directement montées l'une sur l'autre; et
- la figure 6 montre en coupe diamétrale une deuxième variante de réalisation d'un implant comportant deux lentilles.

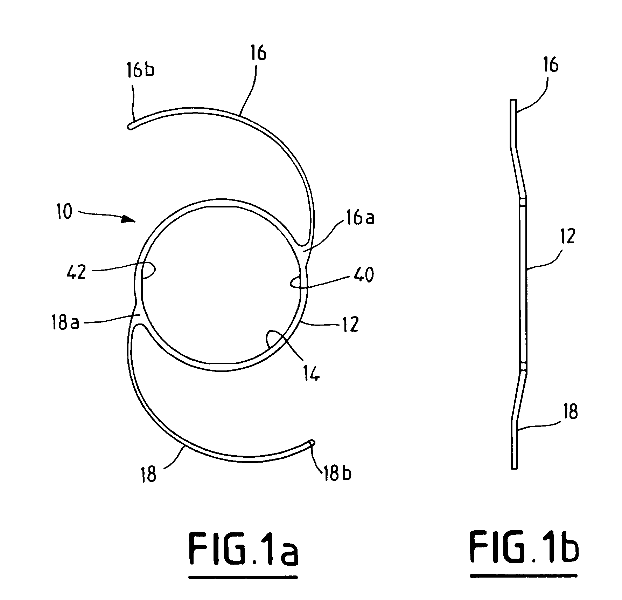

En se référant tout d'abord aux figures 1 à 3, on va décrire un premier mode de réalisation de l'implant intraoculaire dans lequel la partie optique est constituée par une unique lentille par exemple de correction de l'astigmatisme.

Les figures 1a et 1b montrent un mode préféré de réalisation de la partie haptique 10 de l'implant. Celle-ci est constituée par un anneau 12 définissant une ouverture circulaire 14, cet anneau ayant une épaisseur et une largeur réduites. En deux points sensiblement diamétralement opposés de l'anneau 12 sont raccordées deux anses haptiques 16 et 18. La première extrémité des anses 16a, 18a est solidaire de la périphérie de l'anneau 12 et raccordée de préférence par des congés. L'autre extrémité 16b, 18b de chaque anse haptique est libre et vient en appui sur la paroi interne de l'oeil lorsque l'implant est mis en place dans l'oeil. La figure 1a montre un exemple possible de forme d'anse. Cependant toutes les formes habituelles d'anses ou plus généralement de partie haptique peuvent être utilisées à condition qu'elles définissent l'ouverture circulaire 14.

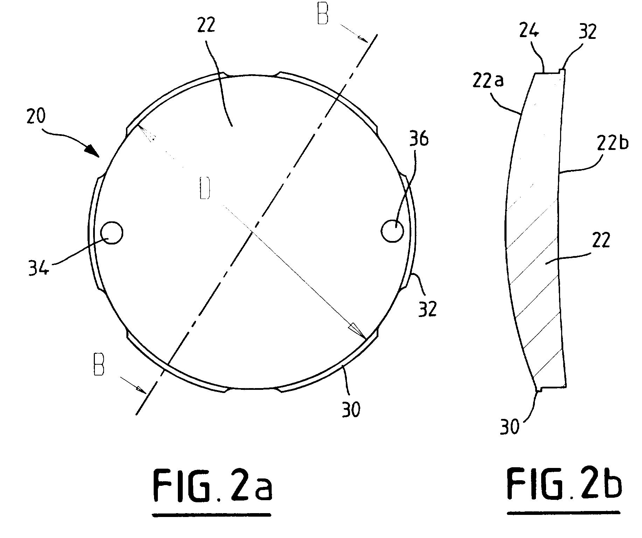

Les figures 2a et 2b montrent un mode préféré de réalisation de la partie optique 20 qui est plus précisément constituée par une lentille 22 dont la périphérie 24 est circulaire et présente un diamètre D égal ou sensiblement égal à celui de l'ouverture 14 de la partie haptique. Sur sa périphérie 24, la lentille 22 présente une première série d'extensions 30 proche de la face avant 22a de la lentille et une deuxième série d'extensions 32 proche de la face arrière 22b de la lentille. La distance entre les extensions 30 et 32 est très légèrement supérieure à l'épaisseur de l'anneau 12. Comme cela a déjà été indiqué, les faces antérieures 22a et postérieures 22b de la lentille 22 sont définies pour corriger par exemple l'astigmatisme en définissant un axe diamétral principal de correction. Dans sa face avant, la lentille 22 est de préférence munie de deux trous 34 et 36 d'orientation de la partie optique 20 par rapport à la partie haptique 10.

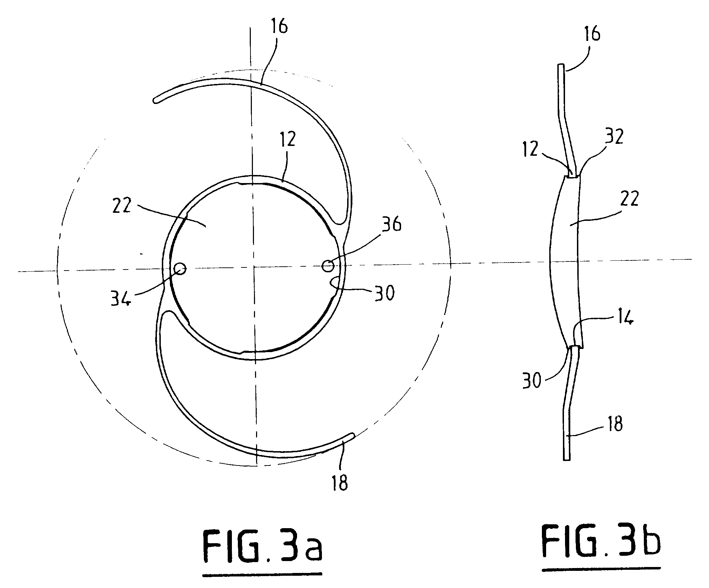

Les figures 3a et 3b montrent l'ensemble de l'implant intraoculaire monté. Pour cela, la lentille 22 est mise en place dans l'anneau 12 de la partie haptique de telle façon que les extensions 30 et 32 soient respectivement en avant et en arrière de l'anneau 12. Ainsi, la lentille 22 est immobilisée en translation selon la direction de son axe optique X X par rapport à la partie haptique mais on peut provoquer sa rotation autour de cet axe par rapport à la partie haptique à l'aide par exemple d'instruments chirurgicaux coopérant avec les trous 34 et 36.

Un autre moyen d'immobilisation en translation consisterait à ménager à la périphérie de la lentille 22 une gorge continue ou discontinue dans laquelle viendrait s'engager le bord interne de l'anneau 12. Il est bien sûr possible de prévoir que l'anneau 12 présente une gorge dans sa face interne et que la périphérie de la lentille soit munie d'extensions radiales pénétrant dans cette gorge.

Afin d'empêcher la libre rotation de la lentille 22 dans la partie haptique de préférence, la face interne de l'anneau 12 définissant l'ouverture 14 présente deux méplats 40 et 42 de faible dimension. Lorsque la lentille est montée dans l'anneau 12, ces méplats réalisent un certain flottement ou freinage qui interdit la rotation de la lentille par rapport à la partie haptique si on n'applique pas à la lentille un couple suffisant pour provoquer sa rotation volontaire.

Une autre solution consiste à donner à l'anneau 12, au repos, une forme non circulaire, par exemple ovale ou elliptique. Après la mise en place de la partie optique qui est circulaire, la déformation résultante de l'anneau permet d'obtenir l'effet de freinage. On peut également prévoir que la périphérie de la lentille ait une forme non rigoureusement circulaire, par exemple ovale ou elliptique, l'anneau 12 ayant au repos une forme circulaire.

Différents matériaux peuvent être utilisés pour réaliser respectivement les parties haptique et optique. Elles peuvent être réalisées en P M M A de façon classique. Si l'on souhaite pouvoir réduire les dimensions de l'incision nécessaire à la mise en place de l'implant dans l'oeil, on peut prévoir que l'anneau 12 soit pliable et que la lentille 22 soit réalisée en un matériau biocompatible souple tel que le gel de silicone ou l'hydrogel. Dans ce cas, bien sûr, la partie haptique est introduite dans un premier temps dans l'oeil puis on introduit la partie optique et le chirurgien doit procéder dans l'oeil au montage de la partie optique sur la partie haptique.

Comme on l'a déjà indiqué, grâce à l'implant qui vient d'être décrit, il est possible, après sa mise en place initiale dans l'oeil, par exemple dans le sac capsulaire, et après que les extrémités libres de la partie haptique se soient solidarisées à la paroi interne de l'oeil, de procéder à la rotation de la lentille pour amener son axe diamétral de correction dans la position angulaire voulue, la partie haptique restant immobile. En d'autres termes, dans le cas de la correction de l'astigmatisme, l'implant qui vient d'être décrit permet d'adapter exactement la position angulaire de correction mais il ne permet pas de modifier ou adapter la puissance de correction.

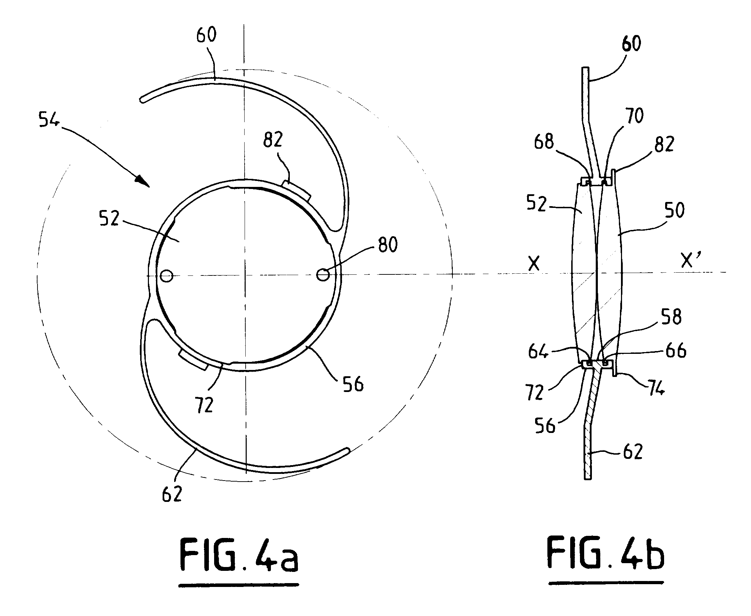

En se référant maintenant aux figures 4a et 4b, on va décrire un deuxième mode de réalisation de l'implant intraoculaire qui permet en outre de modifier la puissance de correction.

La partie optique est constituée par deux lentilles séparées 50 et 52. La partie haptique 54 est constituée, comme dans le cas des figures 1 à 3, par un anneau 56 définissant une ouverture circulaire 58 et par deux boucles haptiques 60 et 62. Les lentilles 50 et 52 sont montées toutes les deux rotatives dans l'ouverture circulaire 58. Pour cela, la face interne de l'anneau 56 peut comporter des gorges annulaires telles que 64 et 66 dans lesquelles on peut faire pénétrer des extensions 68 et 70 respectivement des lentilles 50 et 52. Chaque lentille comporte une deuxième série d'extensions telles que 72 et 74 qui coopère avec les faces externes de l'anneau 56. On obtient ainsi un guidage en rotation séparé de chacune des lentilles et une immobilisation en translation de celles-ci selon la direction de l'axe optique X X'. Pour permettre la rotation séparée de chacune des lentilles, on peut prévoir pour la lentille avant 52, des trous 80. Pour permettre la rotation de la lentille arrière 50, on peut également prévoir des trous sur sa face postérieure ou de préférence deux ergots tels que 82, de préférence munis de trous qui font saillie hors de la face externe de l'anneau 56 de telle manière qu'ils puissent être actionnés par un instrument chirurgical à partir de la chambre antérieure de l'oeil. Dans ce cas encore, il est prévu sur l'anneau des moyens de freinage, non représentés sur les figures, tels que des méplats pour interdire la libre rotation des lentilles en l'absence de sollicitation volontaire. Ces moyens de freinage agissent séparément sur chacune des deux lentilles.

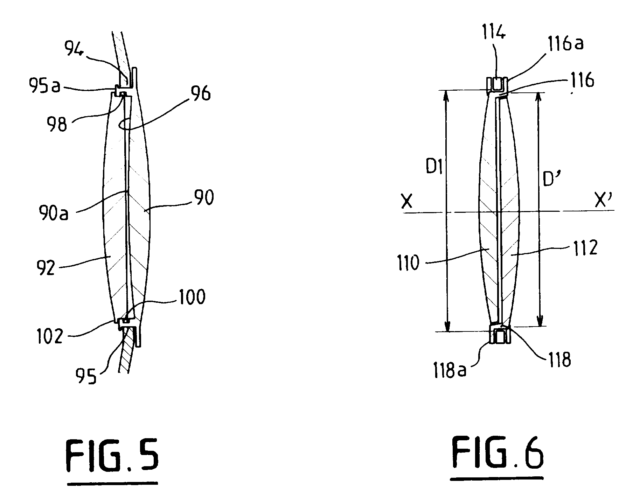

La figure 5 montre une première variante de réalisation d'un implant intraoculaire à deux lentilles 90 et 92. Dans ce mode de réalisation, la lentille 90 est montée dans l'anneau 94 de la partie haptique, comme la lentille 22 des figures 1 à 3. La face antérieure 90a de la lentille 90 est prolongée par une forme en manchon 95 qui définit ainsi un logement sensiblement cylindrique 96. La deuxième lentille 92 est montée à rotation dans ce logement en étant guidée en rotation dans celui-ci. Ce guidage en rotation peut être obtenu par exemple en prévoyant dans le manchon 95 une rainure annulaire 98 dans laquelle peuvent pénétrer des extensions de la lentille 92 référencées 100. Une autre série d'extensions 102 coopère avec la face avant 95a du manchon 95. Des moyens de freinage en rotation séparés sont prévus pour la lentille 90 par rapport à la partie haptique 94 et pour la lentille 92 par rapport à la lentille 90.

Ainsi, en provoquant la rotation globale des deux lentilles, on modifie la position angulaire de la direction diamétrale de correction et en modifiant la position relative des deux lentilles, on peut adapter la puissance de correction.

La figure 6 montre une deuxième variante de réalisation d'un implant à deux lentilles. La lentille avant 110 et la lentille arrière 112 ont un diamètre D' un peu inférieur au diamètre interne D₁ de l'anneau haptique 114. La lentille avant 110 comprend des extensions radiales coudées 116, dont la partie terminale 116a prend appui sur la face arrière de l'anneau 114. De même, la lentille arrière 112 comprend des extensions radiales coudées 118 dont la partie terminale 118a prend appui sur la face avant de l'anneau 114. Ces extensions 116 et 118 correspondent à des angles au centre de valeur limitée. La coopération avec l'anneau 114 des deux séries d'extensions 116 et 118 assurent la solidarisation en translation des lentilles 110 et 112 par rapport à l'anneau 114, tant en autorisant une rotation relative des deux lentilles d'un angle au centre qui dépend essentiellement du nombre d'extensions de chaque lentille.

Il est important de souligner que l'implant pourrait comporter plus de deux lentilles séparées montées rotatives dans la partie haptique. Dans ce cas, il est possible de donner à chaque lentille des surfaces relativement simples à usiner, telles que sphériques, coniques, toriques ou cylindriques, l'empilement de ces lentilles permettant de réaliser une fonction optique globale de correction relativement complexe. Dans ce cas, encore, chaque lentille doit comporter des trous et/ou ergots pour pouvoir provoquer la rotation individuelle de chaque lentille et chaque lentille doit être associée à des moyens de freinage qui lui sont propres.

On comprend que, dans cette invention, quel que soit le mode de réalisation considéré, la partie haptique sert uniquement au maintien de la partie optique dans l'oeil et ne remplit aucune fonction optique. L'intégralité de cette fonction correctrice est réalisée par la partie optique de l'implant.

On comprend d'ailleurs que la partie haptique entoure seulement la partie optique et ne se superpose nullement à elle. Plus précisément, la partie haptique, et notamment l'anneau 12, se trouvent hors du champ de vision de l'oeil dans lequel l'implant est mis en place.

## Revendications

1. Dispositif d'implant intraoculaire pour la correction de l'anisotropie oculaire et notamment de l'astigmatisme comprenant une partie optique ayant une forme sensiblement circulaire et étant apte à corriger l'anisotropie selon une direction diamétrale et une partie haptique, caractérisées en ce que ladite partie optique et ladite partie haptique sont constituées de deux pièces différentes, en ce que ladite partie haptique comprend une première extrémité apte à coopérer avec la paroi interne de l'oeil, lorsque le dispositif d'implant est disposé dans l'oeil, pour maintenir en position ladite partie optique et une deuxième extrémité apte à coopérer avec la périphérie de ladite partie optique pour définir des moyens de guidage en rotation de ladite partie optique autour de son axe optique, des moyens de solidarisation en translation de ladite partie optique par rapport à ladite partie haptique selon la direction de l'axe optique, et des moyens de freinage pour n'autoriser la rotation de ladite partie optique que sous l'effet d'un couple appliqué à ladite partie optique ayant une intensité supérieure à une valeur prédéterminée.

2. Dispositif d'implant intraoculaire selon la revendication 1, caractérisé en ce que ladite deuxième extrémité de la partie haptique est constituée par une pièce de guidage définissant un évidement circulaire dont le diamètre est sensiblement égal à celui de la partie optique, et en ce que la périphérie de la partie optique comporte des moyens formant gorge pour coopérer avec ladite pièce de guidage.

3. Dispositif d'implant intraoculaire selon la revendication 2, caractérisé en ce que les moyens formant gorge comprennent des extensions radiales de la périphérie de la partie optique disposées respectivement de part et d'autre de ladite pièce de guidage selon la direction dudit axe optique.

4. Dispositif d'implant intraoculaire selon la revendication 1, caractérisé en ce que ladite deuxième extrémité de la partie haptique est constituée par une pièce de guidage définissant un évidement circulaire dont le diamètre est sensiblement égal à celui de la partie optique, et en ce que la pièce de guidage comporte des moyens formant gorge pour coopérer avec la périphérie de ladite partie optique.

5. Dispositif d'implant intraoculaire selon l'une quelconque des revendications revendications 2 à 4, caractérisé en ce que ladite pièce de guidage est constituée par une pièce annulaire et en ce que ladite partie haptique comprend en outre au moins deux anses dont les premières extrémités sont libres et incurvées et dont les deuxièmes extrémités sont solidaires de ladite pièce annulaire.

6. Dispositif d'implant intraoculaire selon l'une quelconque des revendications 2 à 5, caractérisé en ce que lesdits moyens de freinage consistent dans le fait que la périphérie externe de ladite pièce de guidage n'est pas circulaire.

7. Dispositif d'implant intraoculaire selon la revendication 6, caractérisé en ce que la périphérie de la pièce de guidage est elliptique ou ovale.

8. Dispositif d'implant intraoculaire selon la revendication 6, caractérisé en ce que la périphérie interne de la pièce de guidage comporte au moins un méplat.

9. Dispositif d'implant intraoculaire selon l'une quelconque des revendications 2 à 5, caractérisé en ce que la périphérie interne de la pièce de guidage est circulaire et en ce que la périphérie de la partie optique n'est pas rigoureusement circulaire.

10. Dispositif d'implant intraoculaire selon l'une quelconque des revendication 1 à 9, caractérisé en ce que ladite partie optique est constituée par une unique lentille dont la périphérie est circulaire.

11. Dispositif d'implant intraoculaire selon la revendication 10, caractérisé en ce que ladite lentille présente sur une de ses faces à proximité de sa périphérie un trou apte à recevoir l'extrémité d'un instrument pour commander la rotation de ladite lentille par rapport à ladite partie haptique.

12. Dispositif selon l'une quelconque des revendications 1 à 9, caractérisé en ce que ladite partie optique est constituée par deux lentilles séparées présentant chacune un axe de correction de l'anisotropie, en ce que lesdits moyens de guidage en rotation et de maintien en translation comprennent des moyens pour guider en rotation séparément les deux lentilles de telle manière que leurs axes de rotation sont communs et confondus avec leurs axes optiques et de maintien en translation desdites deux lentilles, et en ce que des moyens de freinage séparés sont associés à chaque lentille.

13. Dispositif d'implant intraoculaire selon l'une quelconque des revendications 1 à 9, caractérisé en ce que ladite partie optique comprend plus de deux lentilles séparées, en ce que lesdits moyens de guidage en rotation et de maintien en rotation comprennent des moyens pour guider en rotation séparément chaque lentille de telle manière que leurs axes de rotation soient communs et confondus avec leurs axes optiques, et en ce que des moyens de freinage séparés sont associés à chaque lentille.

14. Dispositif d'implant intraoculaire selon l'une quelconque des revendications 10 à 13, caractérisé en ce que chaque lentille comprend des moyens aptes à coopérer avec un instrument chirurgical pour permettre l'application à ladite lentille d'un couple de mise en rotation.

15. Dispositif d'implant intraoculaire selon l'une quelconque des revendications 10 à 14, caractérisé en ce que chaque lentille comporte des surfaces sphériques, coniques, toriques ou cylindriques.
